# EUROPEAN PATENT SPECIFICATION

(11) **EP 2 202 214 B1**
(45) Date of publication and mention of the grant of the patent: **10.01.2018**
(21) Application number: 08831624.5
(22) Date of filing: 29.08.2008
(51) Int. Cl.: C07C 29/145, C07C 31/10, B01J 37/06, B01J 25/00, B01J 25/02

(54) **METHOD FOR PRODUCING ALCOHOL USING AN ACID-TREATED RANEY CATALYST**
VERFAHREN ZUR HERSTELLUNG EINES ALKOHOLS UNTER VERWENDUNG EINES SÄUREBEHANDELTEN RANEY-KATALYSATOS
PROCÉDÉ DE FABRICATION D'ALCOOL UTILISANT UN CATALYSEUR DE RANEY TRAITÉ À L'ACIDE

(30) Priority: 19.09.2007 JP 2007242488
(43) Date of publication of application: 30.06.2010
(73) Proprietor: Mitsui Chemicals, Inc., Tokyo 105-7117 (JP)
(72) Inventor: MORIZANE, Kunihiko, Ichihara-shi Chiba 299-0108 (JP); SHIRAHATA, Tatsuo, Ichihara-shi Chiba 299-0108 (JP); YASUDA, Kozo, Ichihara-shi Chiba 299-0108 (JP)
(74) Representative: J A Kemp
(86) International application number: PCT/JP2008/065503
(87) International publication number: WO 2009/037953

(56) References cited:
- EP-A1- 0 405 956
- EP-A2- 0 379 323
- WO-A1-02/50017
- JP-A- 3 038 536
- JP-A- 3 141 235
- JP-A- 8 323 206
- JP-A- 59 189 938
- US-A- 2 734 921
- US-A- 2 983 734

## Description

### FIELD OF THE INVENTION

The present invention relates to alcohol production processes and acid-treated Raney catalysts. In more detail, the invention relates to processes for producing an alcohol of high purity by reacting a ketone and hydrogen with inhibited by-production of impurities, and relates to acid-treated Raney catalysts used in the processes.

### BACKGROUND OF THE INVENTION

Alcohols are important intermediates in organic synthesis or important solvents in the industry. Use of alcohols has spread in wide-ranged areas, and some applications require high-purity alcohols.

Distillation is generally used for the production of alcohols with high purity, but it involves large amounts of energy. Allowable kinds and amounts of impurities vary depending on the use applications.. In some uses, distillation often cannot provide alcohols of high purity with sufficient level of impurity removal.

Accordingly, reducing impurities that are by-produced in the alcohol-producing reactions is an effective approach for the production of alcohols having high purity.

As known in the art, alcohols are produced by hydrating olefins or hydrogenating the carbonyl groups of ketones.

In the production of isopropyl alcohol for example, olefin hydration methods that are generally used are an indirect hydration method in which propylene is esterified with concentrated sulfuric acid and the resultant ester is hydrolyzed, and a direct hydration method in which propylene is directly hydrated under the catalysis of a heteropoly acid or the like. In these methods, the sulfuric acid or the heteropoly acid used as catalyst corrodes the apparatus. Further, these propylene hydration methods involve by-production of impurities such as n-propyl alcohol, tert-butyl alcohol, 2-methyl-2-pentene and 2-methyl-1-pentene.

The carbonyl groups in ketones are hydrogenated by a reduction method using reagents such as lithium aluminum hydride and sodium boron hydride, or a catalytic reduction method with hydrogen gas.

Exemplary methods with hydrogen gas for the production of isopropyl alcohol include a method using a massive Raney nickel catalyst (Patent Document 1), a method with a gas-liquid-solid three phase trickle bed system (Patent Document 2), and a method in which part of a reaction mixture is circulated to increase the reaction yield (Patent Document 3) .

However, all these methods address the improvement of reaction results and are irrelevant to the quality improvement for the obtainable isopropyl alcohol.

Patent Document 4 discloses a method using hydrogen gas which can produce isopropyl alcohol of high purity even from crude acetone. This method specifies the kinds and amounts of impurities contained in the raw material acetone.
- Patent Document 1:: JP-A-H03-141235
- Patent Document 2:: JP-A-H02-270829
- Patent Document 3:: JP-A-H03-133941
- Patent Document 4:: JP-A-2002-121160

### DISCLOSURE OF THE INVENTION

The present invention has been made in view of the problems in the art as described above. It is therefore an object of the invention to provide processes for producing alcohols such as isopropyl alcohol with high purity and little by-product impurities by reacting a ketone and hydrogen.

The present inventors studied diligently to achieve the above object. They have then found that the object is accomplished by catalytically hydrogenating a ketone under the catalysis of an acid-treated Raney catalyst that is obtained by contact-treating a Raney catalyst with an organic acid. The present invention has been completed based on the finding.

The present invention is concerned with the following.
[1] A process for producing alcohols, comprising catalytically hydrogenating a ketone of Formula (1) below in the presence of a catalyst into an alcohol represented by Formula (2) below,
   the catalyst being an acid-treated Raney catalyst obtainable by contact-treating a Raney catalyst with an organic acid, wherein the contact treatment takes place before the catalytic hydrogenation, and
   wherein the organic acid is at least one organic acid selected from the group consisting of formic acid, acetic acid, propionic acid, butyric acid, valeric acid, oxalic acid, malonic acid, succinic acid, fumaric acid, maleic acid and citric acid; 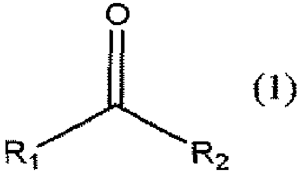 wherein R₁ and R₂ are each independently an alkyl group having the carbon number of 1 to 6; 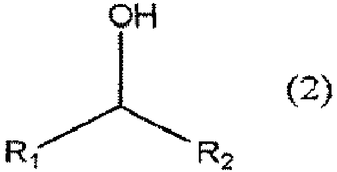 wherein R₁ and R₂ each represent the same group as indicated by R₁ and R₂, respectively, in Formula (1).
[2] The process for producing alcohols according to [1], wherein the acid-treated Raney catalyst is washed with water before the acid-treated Raney catalyst is used in catalytic hydrogenation
[3] The process for producing alcohols according to [1] or [2], wherein the acid-treated Raney catalyst is obtainable by contact-treating a Raney catalyst with an acidic aqueous solution.
[4] The process for producing alcohols according to [3], wherein the acidic aqueous solution has a pH of from 2.5 to less than 7.
[5] The process for producing alcohols according to [3] or [4], wherein the acidic aqueous solution is an aqueous acetic acid solution.
[6] The process for producing alcohols according to any one of [1] to [5], wherein the Raney catalyst is at least one Raney catalyst selected from the group consisting of a Raney nickel and a Raney cobalt.
[7] The process for producing alcohols according to any one of [1] to [6], wherein R₁ and R₂ in Formulae (1) and (2) are each independently a methyl group, an ethyl group, a n-propyl group, an i-propyl group or an i-butyl group.
[8] The process for producing alcohols according to any one of [1] to [7], wherein the ketone of Formula (1) is acetone and the alcohol represented by Formula (2) is isopropyl alcohol.

### ADVANTAGEOUS EFFECTS OF THE INVENTION

The alcohol production processes of the invention use an acid-treated Raney catalyst obtained by contact-treating a Raney catalyst with an organic acid. By the use of the catalyst, alcohols of high purity can be produced with little by-product impurities.

### PREFERRED EMBODIMENTS FOR CARRYING OUT THE INVENTION

The present invention will be described in detail hereinbelow.

### [Alcohol production processes]

In the process for producing alcohols according to the present invention, a ketone of Formula (1) below is catalytically hydrogenated in the presence of a catalyst into an alcohol represented by Formula (2) below. The catalyst is an acid-treated Raney catalyst obtainable by contact-treating a Raney catalyst with an organic acid, selected from the acids as defined above, wherein the contact treatment takes place before the catalytic hydrogenation. 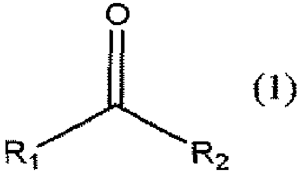 In the formula above, R₁ and R₂ are each independently an alkyl group having the carbon number of 1 to 6.

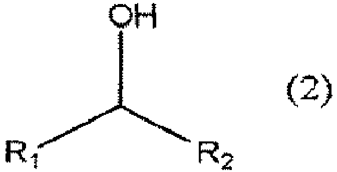

In the formula above, R₁ and R₂ each represent the same group as indicated by R₁ and R₂, respectively, in Formula (1) .

In the compounds having Formulae (1) and (2), the alkyl group having the carbon number of 1 to 6 indicated by R₁ and R₂ include methyl group, ethyl group, n-propyl group, i-propyl group, n-butyl group, i-butyl group, n-pentyl group and n-hexyl group. The groups R₁ and R₂ may be the same or different from each other.

In particular, R₁ and R₂ in Formulae (1) and (2) are preferably each independently a methyl group, an ethyl group, a n-propyl group, an i-propyl group or an i-butyl group. Such compounds have a wide range of industrial applications.

Examples of the ketones represented by Formula (1) include acetone, methyl ethyl ketone, methyl isopropyl ketone and methyl isobutyl ketone. In particular, acetone is preferable.

According to the alcohol production processes of the invention, the alcohols represented by Formula (2) are produced from the ketones as described above. For example, isopropyl alcohol may be produced from acetone, 2-butanol from methyl ethyl ketone, 3-methyl-2-butanol from methyl isopropyl ketone, and 4-methyl-2-pentanol from methyl isobutyl ketone.

According to the alcohol production processes of the invention, isopropyl alcohol having a wide range of industrial applications can be favorably produced from acetone.

The catalysts used in the invention are acid-treated Raney catalysts that are obtainable by contact-treating Raney catalysts with an organic acid, wherein the acid is selected from a group of acids as defined above.

The Raney catalysts in the invention are metal catalysts that are obtained by alloying a metal which is insoluble (hardly soluble) in alkali or acid, e.g., nickel or cobalt, with a metal which is soluble in alkali or acid, e.g., aluminum, silicon, zinc or magnesium, and thereafter dissolving the alkali- or acid-soluble metal from the alloy.

Examples of the Raney catalysts include a Raney nickel, a Raney cobalt, a Raney copper and a Raney iron. From the viewpoint of reaction yield, it is preferable to use at least one Raney catalyst selected from a Raney nickel and a Raney cobalt.

The Raney nickel, Raney cobalt, Raney copper and Raney iron are Raney catalysts in which the metallic composition insoluble (hardly soluble) in alkali or acid is based on nickel, cobalt, copper or iron, respectively.

In the alcohol production processes, the Raney catalyst is contact-treated with an organic acid before the catalytic hydrogenation. The contact treatment may be performed at any time without limitation as long as it takes place before the catalytic hydrogenation. To avoid degradation of the catalyst due to long-term storage, the contact treatment is preferably performed within one day before the reaction, for example immediately before the reaction.

The contact-treated Raney catalysts, namely, the acid-treated Raney catalysts are ignitable. In view of safety, they are preferably stored in water as required.

The acids are organic acids as defined above. The acids may be used singly, or two or more kinds may be used in combination.

The acids used in the contact treatment are usually in the liquid state. Liquid acids may be used, or acids may be dissolved in solvents to give acidic solutions. In view of cost and easy control of acid strength to avoid catalyst deterioration, a preferred acidic solution is an aqueous acidic solution of the acid in water.

The organic acids are selected from formic acid, acetic acid, propionic acid, butyric acid, valeric acid, oxalic acid, malonic acid, succinic acid, fumaric acid, maleic acid and citric acid. Of these, acetic acid is inexpensive and cost effective.

The contact treatment in the invention is not limited as long as the Raney catalyst is brought into contact with the acid. In an exemplary method (I), a cage containing the Raney catalyst is fixed in a container equipped with a stirrer which contains the acid in liquid state such as the acidic aqueous solution, and the Raney catalyst is contact-treated with the acid under mild stirring. According to an exemplary method (II), the method (I) is repeated to a degree that does not deteriorate the Raney catalyst. In an exemplary method (III), the Raney catalyst is packed in a catalytic hydrogenation reactor and the acid in liquid state such as the acidic aqueous solution is supplied thereto in an amount that does not deteriorate the catalyst, thereby continuously contact-treating the Raney catalyst.

Of these contact treatment methods, the acid-treated Raney catalyst can be produced efficiently and good operation properties are obtained by the exemplary method (III) in which the Raney catalyst is packed in a catalytic hydrogenation reactor and the acid in liquid state such as the acidic aqueous solution is supplied thereto in an amount that does not deteriorate the catalyst, thereby continuously contact-treating the Raney catalyst.

The acid-treated Raney catalysts used in the invention are obtained by contact-treating the Raney catalysts with the acids, and preferably by contact-treating the Raney catalysts with the acidic aqueous solution as described above.

The pH of the acidic aqueous solutions used in the contact treatment is not limited as long as it is less than 7. To prevent the deterioration of the catalysts or the corrosion of the apparatus, the pH of the acidic aqueous solutions is preferably from 2.5 to less than 7, and more preferably from 2.5 to 4.

The acidic aqueous solutions used in the contact treatment usually have an acid concentration of 0.001 to 10 mol/L, and preferably 0.01 to 5 mol/L.

The amount of the acidic aqueous solutions used in the contact treatment is not limited as long as the catalysts are not deteriorated. The volume of the acidic aqueous solution is preferably 5 to 200 times, and more preferably 50 to 100 times the volume of the catalyst used.

In the invention, the Raney catalyst may be contact-treated with the acidic aqueous solution at any solution temperature without limitation as long as the temperature is above the freezing point but below the boiling point of the acidic aqueous solution and does not deteriorate the catalyst. The solution temperature is usually in the range of -10°C to 150°C, and preferably 10°C to 80°C. .In view of energy efficiency, the contact treatment is preferably performed at room temperature.

After the Raney catalyst is contact-treated with the acid and before the acid-treated Raney catalyst is used in catalytic hydrogenation, the acid-treated Raney catalyst is preferably washed with water to prevent the acid from decomposing the ketone of Formula (1) or from becoming an impurity in the obtainable alcohol.

The amount of water is not particularly limited, but is preferably 5 to 200 times, and more preferably 50 to 100 times the volume of the acid-treated Raney catalyst.

The washing with water may take place at any temperature without limitation, but is usually carried out at 10°C to 80°C. In view of energy efficiency, the washing is preferably performed at room temperature.

The washing with water may be performed by various methods . In an exemplary method (A), a cage containing the acid-treated Raney catalyst is fixed in a container equipped with a stirrer which contains water, and the acid-treated Raney catalyst is washed with water under mild stirring. According to an exemplary method (B), the method (A) is repeated several times. In an exemplary method (C), the acid-treated Raney catalyst is packed in a catalytic hydrogenation reactor and water is supplied thereto to continuously wash the acid-treated Raney catalyst.

Examples of the reactors for use in the catalytic hydrogenation include packed reactors, trickle bed reactors, multi-tube reactors, moving bed reactors, suspended bubble column reactors and stirring tank reactors. The catalytic hydrogenation may be carried out under conventional conditions.

The reaction temperature and pressure in the catalytic hydrogenation are not particularly limited. These conditions usually vary depending on the atmosphere in the reactor. For example, the temperature and pressure are usually not more than 130°C and in the range of 0.5 to 4 MPa-G in liquid-phase reaction, and are in the range of 60 to 200°C and not more than 1 MPa-G in gas-phase reaction.

In the invention, the molar ratio between the ketone and hydrogen (ketone: hydrogen) used in the catalytic hydrogenation is not particularly limited, but is usually in the range of 1:1 to 1:10. The acid-treated Raney catalyst is typically used in the catalytic hydrogenation in a volume that is 0.1 to 4 times the volume of the ketone.

When the alcohols are produced by the reaction in liquid phase, it is preferable to use reaction solvents from the viewpoint of reaction yield. Examples of the reaction solvents include alcohols such as methanol, ethanol, propanol, butanol and isopropyl alcohol; glycols such as ethylene glycol, propylene glycol, diethylene glycol and triethylene glycol; ethers such as diisopropyl ether, dibutyl ether, ethylene glycol dimethyl ether, diglyme and triglyme; hydrocarbons such as hexane, heptane, cyclohexane and cyclopentane; and water. They may be used singly, or two or more kinds may be used in combination. When the ketone used is acetone, it is preferable that isopropyl alcohol being the obtainable alcohol is used as the solvent because the purification after the alcohol production is facilitated.

According to the processes of the invention, the ketone and hydrogen are reacted under the conditions as described hereinabove to give a high-purity alcohol with little impurities.

The present inventors assume that the alcohol production processes of the invention achieve reduced impurities compared to the conventional processes because the contact-treatment of the Raney catalyst with the acid before the catalytic hydrogenation reduces trace amounts of impurities attached on the Raney catalyst.

In the production of isopropyl alcohol from acetone as the ketone, impurities that are reduced in amount according to the invention are for example 2-methylpentane-2,4-diol, 4-hydroxy-4-methyl-2-pentanone, 4-methyl-2-pentanone and 4-methyl-2-pentanol.

### [Acid-treated Raney catalysts]

The acid-treated Raney catalysts used in the invention are obtained by contact-treating Raney catalysts with acids. The acid-treated Raney catalysts are as described in [Alcohol production processes] hereinabove. The acid-treated Raney catalysts have higher activity compared to conventional Raney catalysts without the contact treatment and are therefore suitably used as hydrogenation catalysts. In detail, the acid-treated Raney catalysts may be used not only in the alcohol production by catalytic hydrogenation of ketones as described above, but also in other production processes such as production of cumene by catalytic hydrogenation of α-methylstyrene and production of methyl isobutyl ketone by catalytic hydrogenation of mesityl oxide.

### EXAMPLES

The present invention will be described in detail hereinbelow.

### [Example 1]

### (Washing of catalyst)

A Raney nickel catalyst (R-200L manufactured by NIKKO RIKA CORPORATION) in a volume of 20 mL was packed in a glass column. At room temperature, an upflow of a 0.1 mol/L aqueous acetic acid solution (pH: 2.9) was supplied to the glass column at a rate of 500 mL/hr for 3 hours. The massive Raney nickel catalyst was thereby contact-treated with the aqueous acetic acid solution to give an acid-treated Raney nickel catalyst.

Subsequently, an upflow of water at room temperature was supplied to the glass column at a rate of 500 mL/hr for 3 hours to wash the acid-treated Raney nickel catalyst.

### (Catalytic hydrogenation of ketone)

A 150 mL stainless steel reactor was packed with 20 mL of the acid-treated Raney nickel catalyst that had been washed with water as described above. Further, 50 mL of acetone, 50 mL of isopropyl alcohol (IPA) and 2 mL of water-were added. The reactor was purged with nitrogen and then with hydrogen. Under the hydrogen atmosphere, the reaction was carried out at a pressure of 0.8 MPa and a temperature of 110°C for 1.5 hours. After the reaction, the reaction liquid was analyzed by gas chromatography. The acetone conversion was 15.8%.

### [Comparative Example 1]

The procedures in Example 1 were repeated except that the Raney nickel catalyst (R-200L manufactured by NIKKO RIKA CORPORATION) was not contact-treated with the aqueous acetic acid solution. The acetone conversion was 13.3%.

The table below shows the contents of impurities other than isopropyl alcohol, acetone and water in the reaction liquids obtained in Example 1 and Comparative Example 1.

### [Table 1]

**Table 1**

| | 2-methylpentane-2,4-diol (ppm) | 4-hydroxy-4-methyl-2-pentanone (ppm) | 4-methyl-2-pentanone (ppm) | 4-methyl-2-pentanol (ppm) | Total (ppm) |
|---|---|---|---|---|---|
| Ex. 1 | 1200 | 860 | 370 | 150 | 2580 |
| Comp. Ex. 1 | 2000 | 1200 | 910 | 440 | 4550 |

| | | | | | |
|---|---|---|---|---|---|
| In Table 1, ppm indicates ppm by weight (relative to the whole reaction liquid at 100 wt%) . | | | | | |

### INDUSTRIAL APPLICABILITY

The alcohol production processes of the invention by reacting ketones with hydrogen can produce alcohols of high purity with little by-product impurities. The acid-treated Raney catalysts of the invention show high hydrogenation activity. The processes and the catalysts of the invention are highly valuable in the industry.

## Claims

1. A process for producing alcohols, comprising catalytically hydrogenating a ketone of Formula (1) below in the presence of a catalyst into an alcohol represented by Formula (2) below,
the catalyst being an acid-treated Raney catalyst obtainable by contact-treating a Raney catalyst with an organic acid,
wherein the contact treatment takes place before the catalytic hydrogenation, and
wherein the organic acid is at least one organic acid selected from the group consisting of formic acid, acetic acid, propionic acid, butyric acid, valeric acid, oxalic acid, malonic acid, succinic acid, fumaric acid, maleic acid and citric acid; 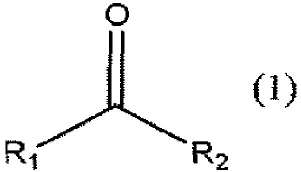 wherein R₁ and R₂ are each independently an alkyl group having the carbon number of 1 to 6; 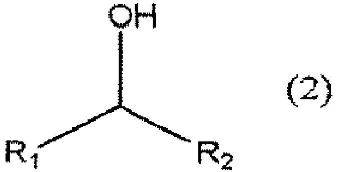 wherein R₁ and R₂ each represent the same group as indicated by R₁ and R₂, respectively, in Formula (1).

2. The process for producing alcohols according to claim 1, wherein the acid-treated Raney catalyst is washed with water before the acid-treated Raney catalyst is used in catalytic hydrogenation.

3. The process for producing alcohols according to claim 1 or 2, wherein the acid-treated Raney catalyst is obtainable by contact-treating a Raney catalyst with an acidic aqueous solution.

4. The process for producing alcohols according to claim 3, wherein the acidic aqueous solution has a pH of from 2.5 to less than 7.

5. The process for producing alcohols according to claim 3 or 4, wherein the acidic aqueous solution is an aqueous acetic acid solution.

6. The process for producing alcohols according to any one of claims 1 to 5, wherein the Raney catalyst is at least one Raney catalyst selected from the group consisting of a Raney nickel and a Raney cobalt.

7. The process for producing alcohols according to any one of claims 1 to 6, wherein R₁ and R₂ in Formulae (1) and (2) are each independently a methyl group, an ethyl group, a n-propyl group, an i-propyl group or an i-butyl group.

8. The process for producing alcohols according to any one of claims 1 to 7, wherein the ketone of Formula (1) is acetone and the alcohol Formula (2) is isopropyl alcohol.

## Patentansprüche

1. Verfahren zur Herstellung von Alkoholen, das eine katalytische Hydrierung eines Ketons der untenstehenden Formel (1) in der Anwesenheit eines Katalysators in einen Alkohol, der durch die untenstehende Formel (2) dargestellt wird, umfasst,
wobei der Katalysator ein säurebehandelter Raney-Katalysator ist, der durch eine Kontaktbehandlung eines Raney-Katalysators mit einer organischen Säure hergestellt werden kann,
wobei die Kontaktbehandlung vor der katalytischen Hydrierung stattfindet, und
wobei die organische Säure mindestens eine organische Säure ist, ausgewählt aus der Gruppe bestehend aus Ameisensäure, Essigsäure, Propionsäure, Buttersäure, Valeriansäure, Oxalsäure, Malonsäure, Bernsteinsäure, Fumarsäure, Maleinsäure und Zitronensäure; 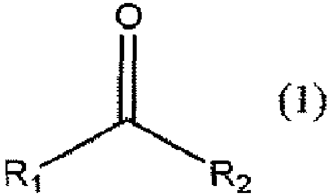 wobei R₁ und R₂ jeweils unabhängig eine Alkylgruppe mit der Kohlenstoffzahl von 1 bis 6 sind; 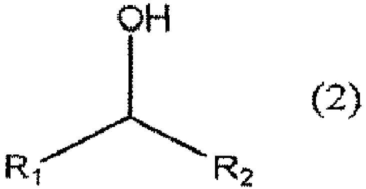 wobei R₁ und R₂ jeweils die gleiche Gruppe wie jeweils durch R₁ und R₂ in Formel (1) angezeigt darstellen.

2. Verfahren zur Herstellung von Alkoholen nach Anspruch 1, wobei der säurebehandelte Raney-Katalysator mit Wasser gewaschen wird, bevor der säurebehandelte Raney-Katalysator in der katalytischen Hydrierung verwendet wird.

3. Verfahren zur Herstellung von Alkoholen nach Anspruch 1 oder 2, wobei der säurebehandelte Raney-Katalysator durch eine Kontaktbehandlung eines Raney-Katalysators mit einer sauren wässrigen Lösung hergestellt werden kann.

4. Verfahren zur Herstellung von Alkoholen nach Anspruch 3, wobei die saure wässrige Lösung einen pH-Wert von 2,5 bis weniger als 7 hat.

5. Verfahren zur Herstellung von Alkoholen nach Anspruch 3 oder 4, wobei die saure wässrige Lösung eine wässrige Essigsäurelösung ist.

6. Verfahren zur Herstellung von Alkoholen nach einem der Ansprüche 1 bis 5, wobei der Raney-Katalysator mindestens ein Raney-Katalysator ist, ausgewählt aus der Gruppe bestehend aus einem Raney-Nickel und einem Raney-Kobalt.

7. Verfahren zur Herstellung von Alkoholen nach einem der Ansprüche 1 bis 6, wobei R₁ und R₂ in den Formeln (1) und (2) jeweils unabhängig eine Methylgruppe, eine Ethylgruppe, eine n-Propylgruppe, eine i-Propylgruppe oder eine i-Butylgruppe sind.

8. Verfahren zur Herstellung von Alkoholen nach einem der Ansprüche 1 bis 7, wobei das Keton der Formel (1) Aceton ist und der Alkohol der Formel (2) Isopropylalkohol ist.

## Revendications

1. Procédé de fabrication d'alcools, comprenant l'hydrogénation catalytique d'une cétone de formule (1) ci-dessous en présence d'un catalyseur en un alcool représenté par la formule (2) ci-dessous ;
le catalyseur étant un catalyseur de Raney traité à l'acide pouvant être obtenu par traitement par contact d'un catalyseur de Raney avec un acide organique,
le traitement par contact ayant lieu avant l'hydrogénation catalytique, et
l'acide organique étant au moins un acide organique choisi dans le groupe constitué par l'acide formique, l'acide acétique, l'acide propionique, l'acide butyrique, l'acide valérique, l'acide oxalique, l'acide malonique, l'acide succinique, l'acide fumarique, l'acide maléique et l'acide citrique ; 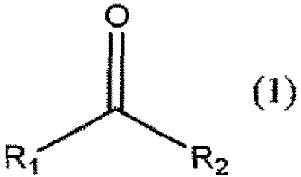 où R₁ et R₂ représentent chacun indépendamment un groupe alkyle présentant le nombre de carbones de 1 à 6 ; 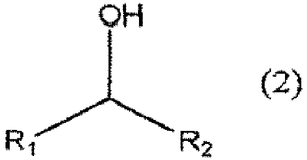 où R₁ et R₂ représentent chacun indépendamment le même groupe tel qu'indiqué par R₁ et R₂, respectivement, dans la formule (1).

2. Procédé de fabrication d'alcools selon la revendication 1, dans lequel le catalyseur de Raney traité à l'acide est lavé à l'eau avant que le catalyseur de Raney traité à l'acide ne soit utilisé dans l'hydrogénation catalytique.

3. Procédé de fabrication d'alcools selon la revendication 1 ou 2, dans lequel le catalyseur de Raney traité à l'acide peut être obtenu par traitement par contact d'un catalyseur de Raney avec une solution aqueuse acide.

4. Procédé de fabrication d'alcools selon la revendication 3, dans lequel la solution aqueuse acide présente un pH allant de 2,5 à moins de 7.

5. Procédé de fabrication d'alcools selon la revendication 3 ou 4, dans lequel la solution aqueuse acide est une solution aqueuse d'acide acétique.

6. Procédé de fabrication d'alcools selon l'une quelconque des revendications 1 à 5, dans lequel le catalyseur de Raney est au moins un catalyseur de Raney choisi dans le groupe constitué par un nickel de Raney et un cobalt de Raney.

7. Procédé de fabrication d'alcools selon l'une quelconque des revendications 1 à 6, dans lequel R₁ et R₂ dans les formules (1) et (2) représentent chacun indépendamment un groupe méthyle, un groupe éthyle, un groupe n-propyle, un groupe i-propyle ou un groupe i-butyle.

8. Procédé de fabrication d'alcools selon l'une quelconque des revendications 1 à 7, dans lequel la cétone de formule (1) est l'acétone et l'alcool de formule (2) est l'alcool isopropylique.
